# EUROPEAN PATENT SPECIFICATION

(11) **EP 0 621 465 B1**
(45) Date of publication and mention of the grant of the patent: **17.09.1997**
(21) Application number: 94103126.2
(22) Date of filing: 02.03.1994
(51) Int. Cl.: G01F 15/18, A61M 16/00, A61B 5/087

(54) **Flow transducer muff**
Stromungsmessermuffe
Manchon pour un débitmètre

(30) Priority: 22.04.1993 SE 9301345
(43) Date of publication of application: 26.10.1994
(73) Proprietor: Siemens-Elema AB, 171 95 Solna 1 (SE)
(72) Inventor: Eriksson, Sture, S-186 36 Vallentuna (SE); Edström, Rolf, S-161 29 Bromma (SE)

(56) References cited:
- DE-A- 2 403 304
- DE-A- 4 034 176

## Description

This invention relates to a flow transducer muff for positioning an essentially square-angled parallelepiped-shaped flow transducer in an expiration channel of a ventilator, comprising a first muff half and a second muff half, devised for connection on both sides of the flow transducer.

Expired gas flow is measured in a flow transducer on the expiration side of a ventilator. The flow transducer is retained in position in the expiration channel with a flow transducer muff which forms a gas-tight connection to the flow transducer. The Operating Manual of the Servo Ventilator 900C, AG 0888 12, August 1988, Siemens-Elema AB, page 10: 2-3, describes one such flow transducer muff. In principle, the known flow transducer muff consists of two halves which are connected on both sides of the flow transducer.

The two muff halves are identical in shape, so there is a risk of the flow transducer being reinstalled in a reversed position in the ventilator after cleaning.

One object of the invention is to achieve a flow transducer muff which assures that the flow transducer is correctly positioned when installed in the expiration channel.

Another object of the invention is to achieve a flow transducer muff which is simple to manufacture, provides a secure, gas-tight connection to the flow transducer, while simultaneously facilitating connection of the flow transducer, and reduces the number of components in the expiration channel.

One such flow transducer muff is achieved in accordance with the invention by a connecting link, attached to the first muff half and to the second muff half so the muff halves can be bent around the connecting link.

With the connecting link, the flow transducer muff consists of a single integrated unit. The connecting link also provides an orientation for the flow transducer, ensuring that it is positioned in the right direction. Since the connecting link is elastically devised, the two muff halves can be readily bent around it when the flow transducer is to be connected to the flow transducer muff or disassembled.

In conjunction herewith, it is advantageous if the connecting link comprises a thin, elastic disc whose width is largely identical to the diameter of the muff halves.

The flow transducer muffs becomes more stable because the connecting link has a width which is generally the same as the diameter of the muff halves.

In order to ensure that the flow transducer really is positioned in the correct direction in the flow transducer muff, it is advantageous if the muff halves is devised with at least one support boss against which at least one of the flow transducer's end surfaces presses when the flow transducer is attached.

Since the flow transducer's opening is not at the center of the flow transducer, the support boss means that the flow transducer can only be connected to the flow transducer muff in one way.

To ensure that the flow transducer muff is correctly placed in the expiration channel, it is advantageous if the muff halves are asymmetrically devised so the flow transducer can only be connected to the expiration channel in one way.

The asymmetry of the muff halves can be achieved either by giving them different shapes or by having different shapes for the connections to other components in the expiration channel.

A refinement of the flow transducer muff is achieved in accordance with the invention in that the muff halves and the connecting link are made in a single piece, preferably by die-casting.

The invention will now be described in greater detail below, referring to three figures in which
FIG. 1 shows one embodiment of the flow transducer muff according to the invention;
FIG. 2 shows a cross-section of the flow transducer muff; and
FIG. 3 shows the flow transducer muff with a connected flow transducer.

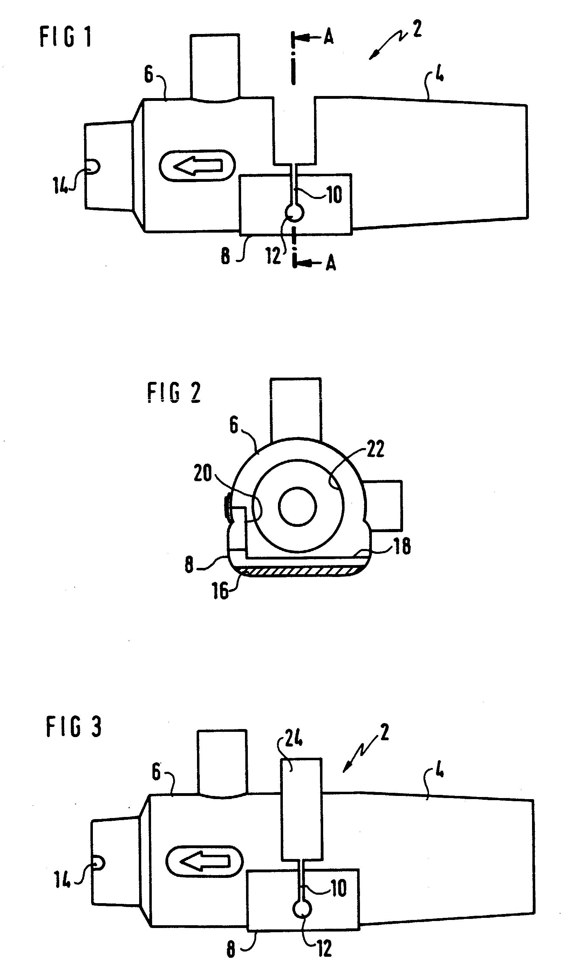

The flow transducer muff 2 in FIG. 1 is die-cast in a single piece and made from an elastic material. The flow transducer muff 2 consists, in principle, of three parts, a first muff half 4, a second muff half 6 and a connecting link 8. The connecting link 8 is provided with a groove 10 which terminates in an open hole 12 to make it possible for the two muff halves 4, 6 to be bent around the connecting link 8.

To ensure that the flow transducer muff 2 is correctly installed in an expiration channel of a ventilator, the muff halves 4, 6 have been devised in different ways at the respective connection end. There is an opening 14 in the end of the second muff halve 6 which fits a corresponding part in the expiration channel so the flow transducer muff 2 makes a correct fit.

In FIG. 2 is shown a cross-section along line A-A on the flow transducer muff 2. Here, it can be clearly seen that the connecting link 8 has a thin disc which holds the two muff halves 4, 6 together.

To ensure that the flow transducer is correctly placed in the flow transducer muff 2, the connecting link 8 has a first support boss 18 and a second support boss 20 against which the flow transducer is pressed when connected to the flow transducer muff 2. Connection of the flow transducer is through a connection opening 22 in the respective muff half 4, 6. Here, the flow transducer is arranged with a corresponding circular shoulder.

FIG. 3 shows the flow transducer muff 2 with a flow transducer 24 connected. Other parts of the flow transducer muff 2 are identical to the parts described in FIG. 1.

## Claims

1. A flow transducer muff for positioning an essentially square-angled parallelepiped-shaped flow transducer (24) in an expiration channel of a ventilator, comprising a first muff half (4) and a second muff half (6), devised for connection to both sides of the flow transducer (24), characterized by an elastic connecting link (8) attached to the first muff half (4) and to the second muff half (6) so the muff halves (4, 6) can be bent around the connecting link (8).

2. A flow transducer muff according to claim 1, characterized in that the connecting link (8) comprises a thin, elastic disc (16) whose width is largely identical to the diameter of the muff halves.

3. A flow transducer muff according to claim 1 or 2, characterized in that the muff halves (4, 6) are devised with at least one support boss (18, 20) against which at least one of the flow transducer's (24) end surfaces presses when the flow transducer is attached.

4. A flow transducer muff according to any of the above claims, characterized in that the muff halves are asymmetrically devised so the flow transducer can only be connected to the expiration channel in one way.

5. A flow transducer muff according to any of the above claims, characterized in that the muff halves (4, 6) and the connecting link (8) are made in a single piece, preferably by die-casting.

## Patentansprüche

1. Eine Flussmessermuffe zum Positionieren eines im wesentlichen quaderförmigen Flussmessers (24) in einem Exspirationskanal eines Ventilators mit einer ersten Muffenhälfte (4) und einer zweiten Muffenhälfte (6), die so ausgebildet sind, dass sie an beide Seiten des Flussmessers (24) anschliessbar sind, **gekennzeichnet durch** ein elastisches Verbindungsglied (8) , das mit der ersten Muffenhälfte (4) und der zweiten Muffenhälfte (6) so verbunden ist, dass die Muffenhälften (4, 6) um das Verbindungsglied (8) herum gebogen werden können.

2. Eine Flussmessermuffe nach Anspruch 1, **dadurch gekennzeichnet**, dass das Verbindungsglied (8) eine dünne, elastische Scheibe (16) aufweist, deren Breite im wesentlichen identisch zu dem Durchmesser der Muffenhälften ist.

3. Eine Flussmessermuffe nach Anspruch 1 oder 2, **dadurch gekennzeichnet**, dass die Muffenhälften (4, 6) mit einem Stützvorsprung (18, 20) versehen sind, gegen den zumindest eine der Endflächen der Flussmesser (24) drückt, wenn der Flussmesser angeschlossen ist.

4. Eine Flussmessermuffe nach einem der vorhergehenden Ansprüche, **dadurch gekennzeichnet**, dass die Muffenhälften derart asymmetrisch ausgebildet sind, dass der Flussmesser nur auf eine Weise mit dem Exspirationskanal verbunden werden kann.

5. Eine Flussmessermuffe nach einem der vorhergehenden Ansprüche, **dadurch gekennzeichnet**, dass die Muffenhälften (4, 6) und das Verbindungsglied (8) als ein Stück hergestellt werden, vorzugsweise durch Formgiessen.

## Revendications

1. Manchon pour débitmètre destiné à positionner un débitmètre (24) de forme sensiblement parallélépipédique à angle droit dans un canal d'expiration d'un ventilateur, comprenant une première moitié (4) de manchon et une seconde moitié (6) de manchon, conçu pour la connexion des deux côtés du débitmètre (24), caractérisé par une liaison (8) de connexion élastique fixée à la première moitié (4) de manchon et à la seconde moitié (6) de manchon de sorte que les moitiés (4, 6) de manchon peuvent être courbées autour de la liaison (8) de connexion.

2. Manchon pour débitmètre suivant la revendication 1, caractérisé en ce que la liaison (8) de connexion comporte un disque (16) élastique mince dont la largeur est dans une grande mesure identique au diamètre des moitiés de manchon.

3. Manchon pour débitmètre suivant la revendication 1 ou 2, caractérisé en ce que les moitiés (4, 6) de manchon sont conçues en ayant au moins une protubérance (18, 20) de support contre laquelle au moins l'une des surfaces d'extrémité du débitmètre (24) exerce une pression lorsque le débitmètre est fixé.

4. Manchon pour débitmètre suivant l'une quelconque des revendications précédentes, caractérisé en ce que les moitiés de manchon sont dissymétriquement conçues de sorte que le débitmètre ne peut être connecté au canal d'expiration que d'une seule manière.

5. Manchon pour débitmètre suivant l'une quelconque des revendications précédentes, caractérisé en ce que les moitiés (4, 6) de manchon et la liaison (8) de connexion sont réalisées en une seule pièce, de préférence par moulage.
